(19) Europäisches Patentamt
European Patent Office
Office européen des brevets

(11) Veröffentlichungsnummer: **0 032 239**
**B1**

(12) **EUROPÄISCHE PATENTSCHRIFT**

(45) Veröffentlichungstag der Patentschrift: **12.01.83**

(21) Anmeldenummer: **80108213.2**

(22) Anmeldetag: **24.12.80**

(51) Int. Cl.³: **C 07 D 249/08,** C 07 D 233/60, C 07 D 233/61, C 07 D 405/06, C 07 D 409/06 // A01N43/50, A01N43/64

(54) **Verfahren zur Herstellung von Azolyl-vinyl-ketonen.**

(30) Priorität: **10.01.80 DE 3000643**

(43) Veröffentlichungstag der Anmeldung: **22.07.81 Patentblatt 81/29**

(45) Bekanntmachung des Hinweises auf die Patenterteilung: **12.01.83 Patentblatt 83/2**

(84) Benannte Vertragsstaaten: **BE CH DE FR GB IT LI NL**

(56) Entgegenhaltungen:
**EP-A-0 002 331**
**EP-A-0 005 600**
**DE-A-2 645 617**
**DE-A-2 652 313**
**DE-A-2 726 043**
**DE-A-2 826 760**
**DE-A-2 838 847**

(73) Patentinhaber: **BAYER AG, Zentralbereich Patente, Marken und Lizenzen, D-5090 Leverkusen 1, Bayerwerk (DE)**

(72) Erfinder: **Elbe, Hans-Ludwig, Dr., Dasnöckel 59, D-5600 Wuppertal 11 (DE)**

EP 0 032 239 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Azolyl-vinyl-ketonen

Die vorliegende Erfindung betrifft ein neues, vorteilhaftes Verfahren zur Herstellung von teilweise bekannten Azolyl-vinyl-ketonen, welche fungizide Eigenschaften aufweisen.

Es ist bereits bekannt geworden, dass man Azolyl-vinyl-ketone der Formel

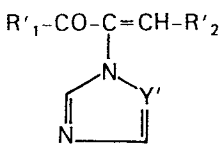

(I)

in welcher
Y′ für ein Stickstoffatom oder die CH-Gruppe steht,
$R'_1$ für gegebenenfalls substituiertes Phenyl und
$R'_2$ für gegebenenfalls substituiertes Phenyl, Alkyl, Furyl oder Thienyl steht (vergleiche DE-OS 2645617 sowie die US-Patentschriften 4067989 und 4086351), oder
$R'_1$ für Alkyl steht und
$R'_2$ für gegebenenfalls substituiertes Phenyl steht (vergleiche die Japanische Patentschrift J5 3130661),

erhält, wenn man Azolyl-ketone der Formel

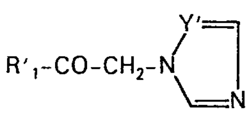

(II)

in welcher
$R'_1$ und Y′ die oben angegebene Bedeutung haben,

mit Aldehyden der Formel

$$O=CH-R'_2 \qquad (III)$$

in welcher
$R'_2$ die oben angegebene Bedeutung hat, in Gegenwart eines üblichen basischen Katalysators, wie z.B. Natriumhydroxid, Natriumcarbonat, Piperidin oder Pyridin, und in Gegenwart eines Lösungsmittels, wie insbesondere aromatische Kohlenwasserstoffe, aber auch Eisessig oder Acetanhydrid, bei Temperaturen zwischen 0 und 120°C umsetzt (vergleiche die o.g. Patentschrift sowie die DE-OS 2838847 und die DE-OS 2645617, in welcher derartige Aldol-Kondensationsreaktionen beschrieben werden).

Dieses Verfahren hat jedoch den Nachteil, dass es hinsichtlich des Substituenten $R'_2$ nicht breit anwendbar ist. Insbesondere sind für aliphatische Aldehyde nur einige ausgewählte Kondensationsprodukte der Formel (I) zugänglich. Ausserdem kommt es häufig, ebenfalls insbesondere beim Einsatz von aliphatischen Aldehyden, zu unerwünschten Doppelbindungsverschiebungen.

Des weiteren sind zur Herstellung von Azolyl-Derivaten andere Verfahren (wie z.B. die Phosphinmethylen-Olefinierungsreaktion oder «Wittig-Reaktion» aus der DE-OS 2652313) und Verfahrensvarianten bekannt geworden, die jedoch teilweise zu anderen Verbindungsklassen führen und zum Teil auch von dem oben erwähnten nächstliegenden Verfahren deutlich verschieden sind (vgl. z.B. DE-OS 2726043 und DE-OS 2826760 und die veröffentlichten EP-Anmeldungen 0002331 und 0005660).

Es wurde gefunden, dass man die teilweise bekannten Azolyl-vinyl-ketone der Formel

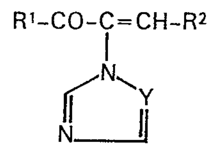

(IV)

in welcher
$R^1$ für Alkyl, mit 1 bis 6 Kohlenstoffatomen, Halogenalkyl mit 1 bis 4 Kohlenstoff- und 1 bis 5 Halogenatomen oder für Phenyl steht, wobei letzteres substituiert sein kann durch Halogen, Alkyl mit 1 bis 4 und Alkoxy mit 1 bis 2 Kohlenstoffatomen, sowie durch gegebenenfalls halogensubstituiertes Phenyl und Phenoxy,
$R^2$ für Alkyl mit 1 bis 12 Kohlenstoffatomen und für Phenyl steht, wobei letzteres substituiert sein kann durch Halogen, Alkyl mit 1 bis 4 und Alkoxy mit 1 bis 2 Kohlenstoffatomen, weiterhin für gegebenenfalls durch Alkyl mit 1 bis 4 Kohlenstoffatomen substituiertes Cycloalkyl mit 3 bis 7 und Cycloalkenyl mit 5 bis 7 Kohlenstoffatomen steht, für Cycloalkylalkyl mit 3 bis 7 Kohlenstoffatomen im Cylcoalkylteil und 1 bis 4 Kohlenstoffatomen im Alkylteil, und ferner für gegebenenfalls durch Halogen und Alkyl mit 1 bis 4 Kohlenstoffatomen substituiertes Furyl oder Thiophenyl steht, und ausserdem für Alkenyl und Alkinyl mit jeweils bis zu 6 Kohlenstoffatomen, wobei die beiden letztgenannten Reste substituiert sein können durch Hydroxy, Alkoxy mit 1 bis 4 Kohlenstoffatomen und durch gegebenenfalls halogensubstituiertes Phenyl, sodann für Indenyl, Fluorenyl und Diphenylmethyl steht, wobei letzteres an den Phenylresten substituiert sein kann durch Halogen, Alkyl mit 1 bis 4 und Alkoxy mit 1 bis 2 Kohlenstoffatomen, und schliesslich für die Gruppierung $CHR^3R^4$ steht, wobei

$R^3$ für Wasserstoff oder Cyano steht und
$R^4$ für Phenyl steht, welches substituiert sein kann durch Halogen, Alkyl mit 1 bis 4 und Alkoxy mit 1 bis 2 Kohlenstoffatomen, und
Y für Stickstoff oder die CH-Gruppe steht,
erhält, wenn man Ketoenamine der Formel

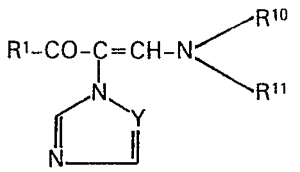

(V)

in welcher
$R^1$ und Y die oben angegebene Bedeutung haben und
$R^{10}$ und $R^{11}$ gleich oder verschieden sind und für Alkyl mit 1 bis 4 Kohlenstoffatomen stehen,
mit metallorganischen Verbindungen der Formel

$Z-R^2$ (VI)

in welcher
$R^2$ die oben angegebene Bedeutung hat, und
Z für die Gruppe Hal-Mg oder für ein Alkalimetall- oder die Gruppierung $LiCuR^2$ steht, wobei
Hal für Halogen steht und
$R^2$ die oben angegebene Bedeutung hat,
in Gegenwart eines Lösungsmittels umsetzt.

Die Verbindungen der Formel (IV) können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an.

Es ist als ausgesprochen überraschend zu bezeichnen, dass die erfindungsgemässe Umsetzung in der angegebenen Art und Weise abläuft. Die sonst häufig zu erwartende und beobachtete Reaktion der metallorganischen Verbindungen mit der Carbonylgruppe des Ketoenamins unterbleibt. Es ist gleichfalls überraschend, dass unerwünschte Doppelbindungsverschiebungen unterbleiben.

Das erfindungsgemässe Verfahren weist eine Reihe von Vorteilen auf. So sind alle Verbindungen sowohl aus der Triazol- als auch aus der Imidazol-Reihe in guten Ausbeuten zugänglich, wobei es keine Einschränkung hinsichtlich des Substituenten $R^2$ gibt. Unerwünschte Doppelbindungsverschiebungen, wie sie bei bekannten Aldolreaktionen, insbesondere mit aliphatischen Aldehyden, häufiger zu beobachten sind, unterbleiben, was zu einer einfacheren Isolierung und einer höheren Ausbeute an gewünschtem Produkt der Formel (IV) führt.

Die nach dem erfindungsgemässen Verfahren herstellbaren Azolylvinyl-ketone sind durch die Formel (IV) allgemein definiert.

Verwendet man zu deren Herstellung beispielsweise 1-(2,4-Dichlorphenyl)-3-dimethylamino-2-(1,2,4-triazol-1-yl)-prop-2-en-1-on und tert.-Butyl-magnesium-bromid als Ausgangsstoffe, so kann der Reaktionsablauf des erfindungsgemässen Verfahrens durch das folgende Formelschema wiedergegeben werden:

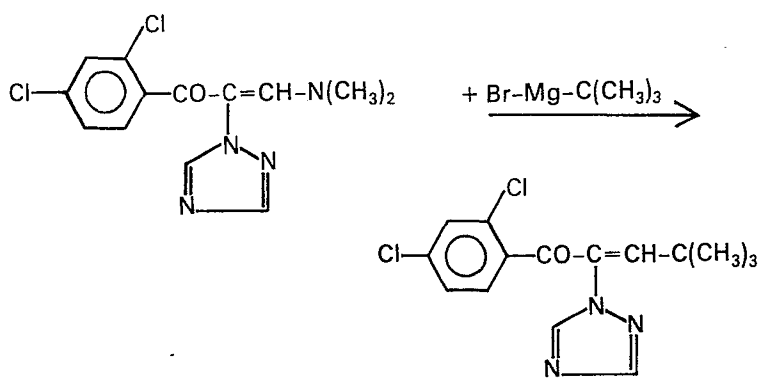


Die bei der Durchführung des erfindungsgemässen Verfahrens als Ausgangsstoffe benötigten Ketoenamine sind durch die Formel (V) allgemein definiert.

Die Verbindungen sind noch nicht bekannt. Sie werden erhalten, indem man Azolyl-ketone der Formel

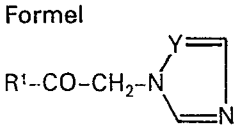

(VII)

in welcher
$R^1$ und Y die oben angegebene Bedeutung haben,
mit Amidacetalen bzw. Aminalestern der Formeln

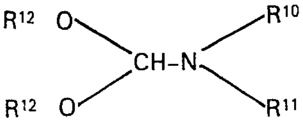

(VIIIa)

bzw.

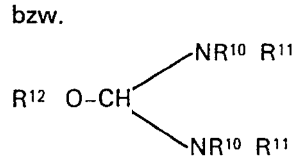

(VIIIb)

in welchen
$R^{10}$ und $R^{11}$ die oben angegebene Bedeutung haben und
$R^{12}$ für Alkyl mit 1 bis 4 Kohlenstoffatomen steht,
in an sich bekannter Art und Weise in Gegenwart eines inerten organischen Lösungsmittels, wie beispielsweise aromatische Kohlenwasserstoffe und wie insbesondere ein Überschuss an eingesetztem Amidacetal bzw. Aminalester der Formel (VIIIa) bzw. (VIIIb), in der Siedehitze umsetzt [vergleiche hierzu auch Chem. Ber. 101, 41–50 (1968); J. Org. Chem. 43, 4248–50 (1978) sowie die Herstellungsbeispiele].

Die Azolyl-ketone der Formel (VII) sind bekannt [vergleiche DE-OS 2431407 (LeA 15735), DE-OS 2610022 und DE-OS 2638470). Sie lassen sich nach üblichen Methoden herstellen, indem man die entsprechenden Halogen-ketone in Gegenwart eines Säurebinders mit 1,2,4-Triazol oder Imidazol umsetzt.

Die Amidacetale bzw. Aminalester der Formeln (VIIIa) bzw. (VIIIb) sind allgemein bekannte Verbindungen der organischen Chemie [vergleiche z.B. Chem. Ber. 101, 41-50 (1968) und J. Org. Chem. 43, 4248-50 (1978)].

Die ausserdem für die erfindungsgemässe Umsetzung als Ausgangsstoffe zu verwendenden metallorganischen Verbindungen sind durch die Formel (VI) allgemein definiert. In dieser Formel steht $R^2$ für diejenigen Reste, die bereits im Zusammenhang mit der Beschreibung der erfindungsgemäss herstellbaren Stoffe der Formel (IV) für diesen Substituenten genannt wurden. Z steht vorzugsweise für die Gruppe Hal-Mg, wobei Hal vorzugsweise für Chlor doer Brom steht. Dies bedeutet, dass als metallorganische Verbindungen der Formel (VI) vorzugsweise die sogenannten «Grignard-Verbindungen» verwendet werden, welche allgemein bekannte Verbindungen der organischen Chemie sind.

An metallorganischen Verbindungen der Formel (VI) können gegebenenfalls Kupfer-Komplexe der allgemeinen Formel

$Li^{(+)} [Cu(R^2)_2]^{(-)}$

Verwendung finden.

Als Lösungsmittel kommen für die erfindungsgemässe Umsetzung vorzugsweise inerte organische Lösungsmittel in reiner Form oder im Gemisch in Frage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei der Durchführung des erfindungsgemässen Verfahrens in einem grösseren Bereich variiert werden. Im allgemeinen arbeitet man zwischen −50 und +150°C, vorzugsweise zwischen −20 und +120°C.

Bei der Durchführung des erfindungsgemässen Verfahrens setzt man auf 1 Mol Ketoenamin der Formel (V) vorzugsweise 1 bis 1,5 Mol an metallorganischer Verbindung der Formel (VI) ein. Die Isolierung der Verbindungen der Formel (IV) erfolgt in üblicher Weise.

Die erfindungsgemäss herstellbaren Wirkstoffe der Formel (IV) zeichnen sich bekanntermassen durch sehr gute fungizide Wirksamkeit aus (vergleiche DE-OS 2645617, US-Patentschriften 4067989 und 4086351 sowie die Japanische Patentschrift J5 3130661).

Das erfindungsgemässe Verfahren sei anhand der folgenden Herstellungsbeispiele erläutert.

Herstellungsbeispiele

Beispiel 1

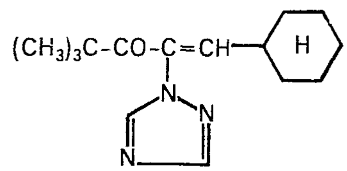

11,1 g (0,05 Mol) 4,4-Dimethyl-1-dimethylamino-2-(1,2,4-triazol-1-yl)-pent-1-en-3-on werden in 250 ml Ether gelöst. Dazu tropft man innerhalb von 30 Minuten eine etherische Lösung von 13,1 g (0,07 Mol) Cyclohexylmagnesiumbromid. Man lässt eine Stunde nachrühren, versetzt das Reaktionsgemisch mit 250 ml Wasser und stellt mit Salzsäure auf einen pH-Wert von 6-7 ein. Die organische Phase wird abgetrennt, getrocknet, filtriert und eingeengt. Man erhält 10,8 g (83% der Theorie) 1-Cyclohexyl-4,4-dimethyl-2-(1,2,4-triazol-1-yl)-pent-1-en-3-on vom Brechungsindex $n_D^{20} = 1,5003$.

In analoger Weise werden die folgenden Verbindungen, der allgemeinen Formel

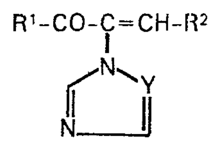 (IV)

hergestellt:

| Bsp. Nr. | $R^1$ | $R^2$ | Y | Schmelzpunkt (°C) |
|---|---|---|---|---|
| 2 | $(CH_3)_3C$ | 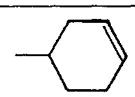 | N | 40-48 |
| 3 | $(CH_3)_3C$ | 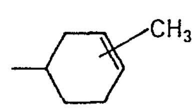 | N | 49 |
| 4 | $(CH_3)_3C$ | 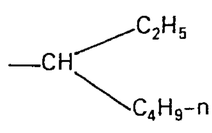 | N | Öl |

| Beispiel Nr. | $R^1$ | $R^2$ | Y | Schmelzpunkt (°C) |
|---|---|---|---|---|
| 5 | $ClCH_2-C(CH_3)_2-$ | 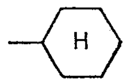 | N | 51 |
| 6 | $ClCH_2-C(CH_3)_2-$ | 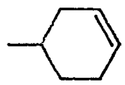 | N | Öl |
| 7 | $FCH_2-C(CH_3)_2-$ | 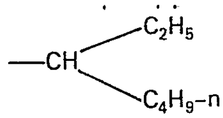 | N | Öl |
| 8 | $FCH_2-C(CH_3)_2-$ | 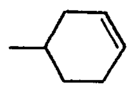 | N | Öl |
| 9 | $FCH_2-C(CH_3)_2-$ | 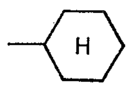 | N | Öl |
| 10 | 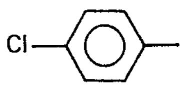 | $n-C_3H_7$ | N | Öl |
| 11 | 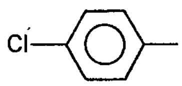 | 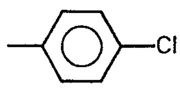 | N | 185–86 |
| 12 | 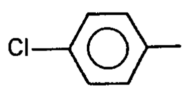 | 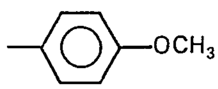 | N | 80–82 |
| 13 | 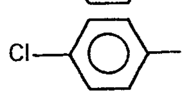 | 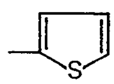 | N | 110–11 |
| 14 | 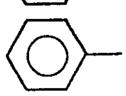 | 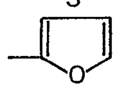 | N | 108–10 |
| 15 | 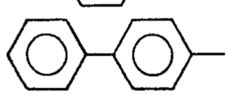 | 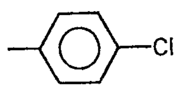 | N | 171–73 |
| 16 | 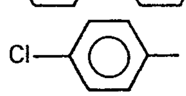 | 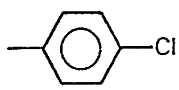 | CH | 117–20 |
| 17 | 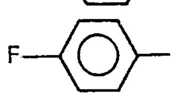 | 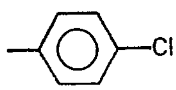 | CH | 142–44 |
| 18 | 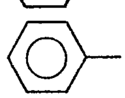 | 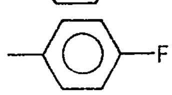 | CH | 85–86 |
| 19 | 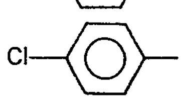 | 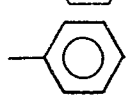 | CH | 146–49 |
| 20 | $(CH_3)_3C-$ | 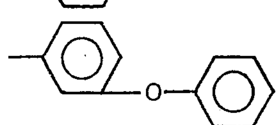 | CH | 88–90 |
| 21 | $(CH_3)_3C-$ | 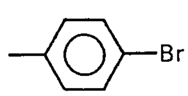 | CH | 90–92 |
| 22 | $(CH_3)_3C-$ | 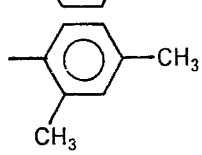 | CH | 65–70 |

| Beispiel Nr. | $R^1$ | $R^2$ | Y | Schmelzpunkt (°C) |
|---|---|---|---|---|
| 23 | $(CH_3)_3C-$ | 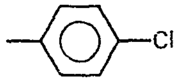 | N | 68 |
| 24 | $(CH_3)_3C-$ | 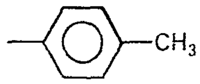 | N | 65 |
| 25 | $(CH_3)_3C-$ | 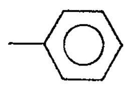 | N | $n_D^{20}$ = 1,557 |
| 26 | $(CH_3)_3C-$ | 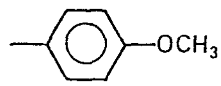 | N | 75 |
| 27 | $(CH_3)_3C-$ | 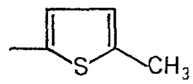 | N | 95 |
| 28 | $(CH_3)_3C-$ | 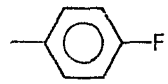 | N | $n_D^{20}$ = 1,5560 |
| 29 | $(CH_3)_3C-$ | 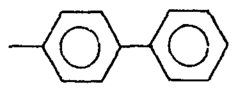 | N | 118 |
| 30 | $(CH_3)_3C-$ | 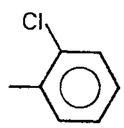 | CH | 90 |
| 31 | 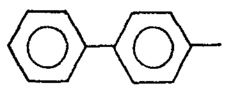 | 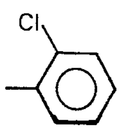 | CH | 110 |

Herstellung der neuen Ketoenamine der Formel (V)

Beispiel V-1

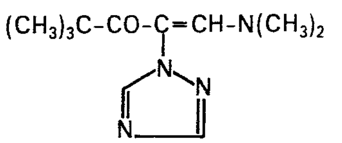

250,8 g (1,5 Mol) 3,3-Dimethyl-1-(1,2,4-triazol-1-yl)-butan-2-on werden mit 196 g (1,65 Mol) Dimethylformamiddimethylacetal 5 Stunden unter Rückfluss erhitzt. Danach wird das überschüssige Acetal abdestilliert. Man erhält 306 g (92% der Theorie) 4,4-Dimethyl-1-dimethylamino-2-(1,2,4-triazol-1-yl)-pent-1-en-3-on vom Brechungsindex $n_D^{20}$= 1,531.

Herstellung des Ausgangsproduktes

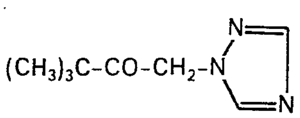

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man lässt 5 Stunden unter Rückfluss rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62–64°C.

Beispiel V-2

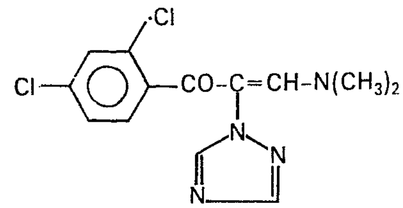

266 g (1 Mol) ω-(1,2,4-Triazol-1-yl)-2,4-dichloracetophenon werden mit 131 g (1,1 Mol) Dimethylformamid-dimethylacetal 5 Stunden unter Rückfluss erhitzt. Danach wird das überschüssige Acetal abdestilliert. Das zurückbleibende Öl kristallisiert beim Abkühlen. Man erhält 292 g

(94% der Theorie) 3-(2,4-Dichlorphenyl)-1-dimethylamino-2- (1,2,4-triazol-1-yl) -prop-1-en-3-on vom Schmelzpunkt 173°C.

Beispiel V-3

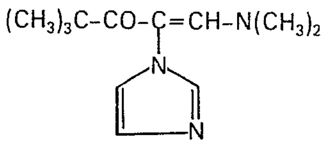

41,6 g (0,25 Mol) 3,3-Dimethyl-1-(imidazol-1-yl)-butan-2-on werden mit 35,7 g (0,3 Mol) Dimethylformamid-dimethylacetal 5 Stunden unter Rückfluss erhitzt. Danach wird das überschüssige Acetal abdestilliert. Das zurückbleibende Öl kristallisiert beim Abkühlen. Man erhält 50 g (90,5% der Theorie) 4,4-Dimethyl-1-dimethylamino-2-(imidazol-1-yl)-pent-1-en-3-on vom Schmelzpunkt 45–50°C.

In entsprechender Weise werden die anderen Ketoenamine der Formel (V) erhalten.

**Patentansprüche**

1. Verfahren zur Herstellung von Azolyl-vinylketonen der Formel

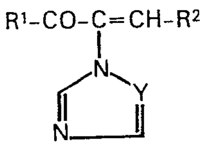

(IV)

in welcher

$R^1$ für Alkyl mit 1 bis 6 Kohlenstoffatomen, Halogenalkyl mit 1 bis 4 Kohlenstoff- und 1 bis 5 Halogenatomen oder für Phenyl steht, wobei letzteres substituiert sein kann durch Halogen, Alkyl mit 1 bis 4 und Alkoxy mit 1 bis 2 Kohlenstoffatomen sowie durch gegebenenfalls halogensubstituiertes Phenyl und Phenoxy,

$R^2$ für Alkyl mit 1 bis 12 Kohlenstoffatomen und für Phenyl steht, wobei letzteres substituiert sein kann durch Halogen, Alkyl mit 1 bis 4 und Alkoxy mit 1 bis 2 Kohlenstoffatomen, weiterhin für gegebenenfalls durch Alkyl mit 1 bis 4 Kohlenstoffatomen substituiertes Cycloalkyl mit 3 bis 7 und Cycloalkenyl mit 5 bis 7 Kohlenstoffatomen steht, für Cycloalkylalkyl mit 3 bis 7 Kohlenstoffatomen im Cycloalkylteil und 1 bis 4 Kohlenstoffatomen im Alkylteil, und ferner für gegebenenfalls durch Halogen und Alkyl 1 bis 4 Kohlenstoffatomen substituiertes Furyl oder Thiophenyl steht und ausserdem für Alkenyl und Alkinyl mit jeweils bis zu 6 Kohlenstoffatomen, wobei die beiden letztgenannten Reste substituiert sein können durch Hydroxy, Alkoxy mit 1 bis 4 Kohlenstoffatomen und durch gegebenenfalls halogensubstituiertes Phenyl, sodann für Indenyl, Fluorenyl und Diphenylmethyl steht, wobei letzteres an den Phenylresten substituiert sein kann durch Halogen, Alkyl mit 1 bis 4 und Alkoxy mit 1 bis 2 Kohlenstoffatomen, und schliesslich für die Gruppierung $CHR^3R^4$ steht, wobei

$R^3$ für Wasserstoff oder Cyano steht und

$R^4$ für Phenyl steht, welches substituiert sein kann durch Halogen, Alkyl mit 1 bis 4 und Alkoxy mit 1 bis 2 Kohlenstoffatomen, und

Y für Stickstoff oder die CH-Gruppe steht,

dadurch gekennzeichnet, dass man Ketoenamine der Formel

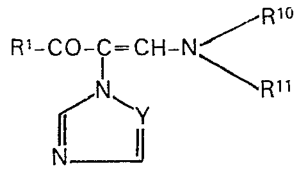

(V)

in welcher

$R^1$ und Y die oben angegebene Bedeutung haben und

$R^{10}$ und $R^{11}$ gleich oder verschieden sind und für Alkyl mit 1 bis 4 Kohlenstoffatomen stehen,

mit metallorganischen Verbindungen der Formel

$$Z-R^2 \qquad (VI)$$

in welcher

$R^2$ die oben angegebene Bedeutung hat und

Z für die Gruppe Hal-Mg oder für ein Alkalimetall- oder die Gruppierung $LiCuR^2$ steht, wobei

Hal für Halogen steht und

$R^2$ die oben angegebene Bedeutung hat,

in Gegenwart eines Lösungsmittels umsetzt.

2. Verfahren gemäss Anspruch 1, dadurch gekennzeichnet, dass man die Umsetzung im Temperaturbereich zwischen −50 und +150°C vornimmt.

3. Verfahren gemäss Ansprüchen 1 und 2, dadurch gekennzeichnet, dass man die Umsetzung im Temperaturbereich zwischen −20 und +120°C vornimmt.

4. Verfahren gemäss Anspruch 1, dadurch gekennzeichnet, dass man die Umsetzung in einem inerten organischen Lösungsmittel vornimmt.

5. Ketoenamine der Formel

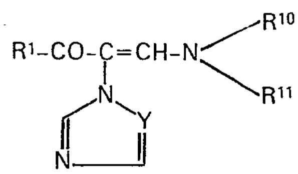

(V)

in welcher

$R^1$ und Y die in Anspruch 1 angegebene Bedeutung haben und

$R^{10}$ und $R^{11}$ gleich oder verschieden sind und für Alkyl mit 1 bis 4 Kohlenstoffatomen stehen.

**Claims**

1. Process for the preparation of azolyl-vinyl ketones of the formula

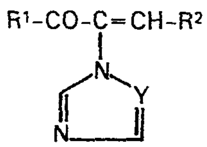

(IV)

in which

$R^1$ represents alkyl with 1 to 6 carbon atoms, halogenoalkyl with 1 to 4 carbon atoms and 1 to 5 halogen atoms or phenyl, it being possible for the latter to be substituted by halogen, alkyl with 1 to 4 carbon atoms and alkoxy with 1 to 2 carbon atoms as well as by optionally halogen-substituted phenyl and phenoxy,

$R^2$ represents alkyl with 1 to 12 carbon atoms and phenyl, it being possible for the latter to be substituted by halogen, alkyl with 1 to 4 carbon atoms and alkoxy with 1 to 2 carbon atoms, furthermore cycloalkyl with 3 to 7 carbon atoms and cycloalkenyl with 5 to 7 carbon atoms which are optionally substituted by alkyl with 1 to 4 carbon atoms, cycloalkylalkyl with 3 to 7 carbon atoms in the alkyl part and 1 to 4 carbon atoms in the alkyl part, and also furyl or thiophenyl which are optionally substituted by halogen and alkyl with 1 to 4 carbon atoms and in addition alkenyl and alkinyl within each case up to 6 carbon atoms, it being possible for the two last-mentioned radicals to be substituted by hydroxyl, alkoxy with 1 to 4 carbon atoms and by optionally halogen-substituted phenyl, then indenyl, fluorenyl and diphenylmethyl, it being possible for the phenyl radicals of the latter to be substituted by halogen, alkyl with 1 to 4 carbon atoms and alkoxy with 1 to 2 carbon atoms, and finally the grouping $CHR^3R^4$, wherein

$R^3$ represents hydrogen or cyano and

$R^4$ represents phenyl which can be substituted by halogen, alkyl with 1 to 4 carbon atoms and alkoxy with 1 to 2 carbon atoms, and

Y represents nitrogen or the CH group,

characterised in that keto-enamines of the formula

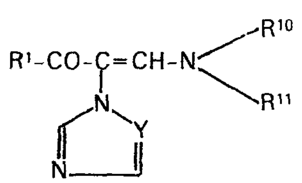

(V)

in which

$R^1$ and Y have the meaning indicated above and

$R^{10}$ and $R^{11}$ are identical or different and represent alkyl with 1 to 4 carbon atoms,

are reacted with organometallic compounds of the formula

$$Z\text{-}R^2 \qquad (VI)$$

in which

$R^2$ has the meaning indicated above and

Z represents the group Hal-Mg or an alkali metal or the grouping $LiCuR^2$,

wherein

Hal represents halogen and

$R^2$ has the meaning indicated above,

in the presence of a solvent.

2. Process according to Claim 1, characterised in that the reaction is carried out in the temperature range between −50 and +150°C.

3. Process according to Claims 1 and 2, characterised in that the reaction is carried out in the temperature range between −20 and +120°C.

4. Process according to Claim 1, characterised in that the reaction is carried out in an inert organic solvent.

5. Keto-enamines of the formula

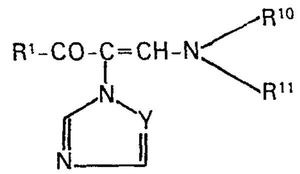

(V)

in which

$R^1$ and Y have the meaning indicated in Claim 1 and

$R^{10}$ and $R^{11}$ are identical or different and represent alkyl with 1 to 4 carbon atoms.

**Revendications**

1. Procédé pour la fabrication d'azolylvinylcétones de formule

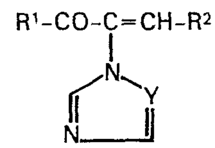

(IV)

dans laquelle

$R^1$ représente un reste alkyle en $C_1$-$C_6$, halogénoalkyle en $C_1$-$C_4$ et ayant 1 à 5 atomes d'halogène ou un reste phényle, ce dernier pouvant être substitué par halogène, alkyle en $C_1$-$C_4$ et alcoxy en $C_1$-$C_2$, ainsi que par phényle éventuellement halogéno-substitué et phénoxy,

$R^2$ représente un reste alkyle en $C_1$-$C_{12}$ ou phényle, ce dernier pouvant être substitué par halogène, alkyle en $C_1$-$C_4$, et alcoxy en $C_1$-$C_2$, en outre un reste cycloalkyle en $C_3$-$C_7$ substitué par alkyle en $C_1$-$C_4$, et un reste cycloalcényle en $C_5$-$C_7$, un reste (cycloalkyl en $C_3$-$C_7$)alkyle en $C_1$-$C_4$, et en outre un reste furyle ou thiophényle substitué par halogène et alkyle en $C_1$-$C_4$ et en outre un reste alcényle et alcynyle, chacun jusqu'en $C_6$, ces deux derniers restes pouvant être substitués par hydroxy, alcoxy en $C_1$-$C_4$ et par phényle éventuellement halogéno-substitué, ensuite des restes indényle, fluorényle et diphénylméthyle, ce dernier pouvant être substitué sur les restes phényle par halogène, alkyle en $C_1$-$C_4$ et alcoxy en $C_1$-$C_2$, et enfin le gorupement $CHR^3R^4$ dans lequel

$R^3$ représente l'hydrogène ou un reste cyano et

$R^4$ représente un groupe phényle qui peut être substitué par halogène, alkyle en $C_1$-$C_4$ et alcoxy en $C_1$-$C_2$ et

Y représente l'azote ou le groupe CH,

caractérisé en ce que l'on fait réagir des cétoèneamines de formule

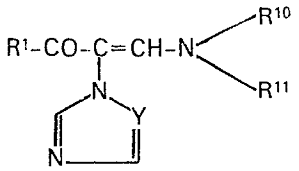

(V)

dans laquelle
$R^1$ et Y ont la signification indiquée ci-dessus et
$R^{10}$ et $R^{11}$ sont identiques ou différents et représentent chacun un reste alkyle en $C_1$–$C_4$,
avec des composés organométalliques de formule

$Z$–$R^2$ (VI)

dans laquelle
$R^2$ a la signification indiquée ci-dessus et
Z représente le groupe Hal–Mg ou un métal alcalin ou le groupement $LiCuR^2$, dans lesquels
Hal représente un halogène et
$R^2$ a la signification indiquée ci-dessus,
en présence d'un solvant.

2. Procédé selon la revendication 1, caractérisé en ce que l'on effectue la réaction dans un domaine de température entre −50 et +120°C.

3. Procédé selon les revendications 1 et 2, caractérisé en ce que l'on effectue la réaction dans le domaine de température entre −20 et +120°C.

4. Procédé selon la revendication 1, caractérisé en ce que l'on effectue la réaction dans un solvant organique inerte.

5. Cétoèneamines de formule

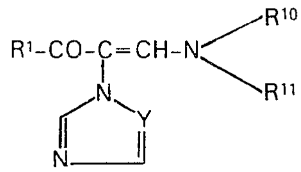

(V)

dans laquelle
$R^1$ et Y ont la signification indiquée dans la revendication 1 et
$R^{10}$ et $R^{11}$ sont identiques ou différents et représentent chacun un groupe alkyle en $C_1$–$C_4$.